# FASCICULE DE BREVET EUROPEEN

(11) **EP 3 167 279 B1**
(45) Date de publication et mention de la délivrance du brevet: **08.04.2020**
(21) Numéro de dépôt: 15736461.3
(22) Date de dépôt: 08.07.2015
(51) Int. Cl.: G01N 21/85, B07C 5/342, G01N 15/14, G01N 33/02, G01N 21/84

(54) **UNITÉ D'ANALYSE DE GRAINS NON COMPLETEMENT OPAQUES**
EINHEIT ZUR ANALYSE VON TEILWEISE OPAKEN KÖRNERN
UNIT FOR ANALYZING PARTIALLY OPAQUE GRAINS

(30) Priorité: 09.07.2014 FR 1456609
(43) Date de publication de la demande: 17.05.2017
(73) Titulaire: Optomachines, 63200 Riom (FR)
(72) Inventeur: PARADIS, François, F-63200 Riom (FR)
(74) Mandataire: Cabinet Laurent & Charras
(86) Numéro de dépôt international: PCT/EP2015/065644
(87) Numéro de publication internationale: WO 2016/005475

(56) Documents cités:
- WO-A1-98/30886
- FR-A1- 2 949 984
- JP-A- 2003 098 096
- US-A- 5 835 206

## Description

L'invention concerne le domaine des appareils optiques d'observation et d'inspection. Elle concerne plus particulièrement une unité d'analyse de la vitrosité de graines et grains non complètement opaques, c'est à dire partiellement ou totalement vitreux, comme le blé dur, le riz blanchi ou le riz paddy.

Le problème à la base de l'invention est de faciliter l'analyse qualité des blés durs et des riz blanchis en améliorant le contraste des grains avec le fond éclairé.

Actuellement les analyses sont faites visuellement avec des appareils de laboratoire, alimentés manuellement et avec de mauvaises conditions d'éclairement. En particulier pour le blé dur, les conditions d'analyse ne permettent que difficilement de distinguer les grains mitadinés. Or, l'on sait que plus l'observation est facile, plus fiable et rapide est l'interprétation humaine.

Pour le riz, cela permet de distinguer la caractéristique que l'on appelle la « perle », c'est à dire une couleur blanche plus opaque à l'intérieur du grain.

Cette unité d'analyse peut aussi servir à l'analyse d'autres matériaux plus ou moins translucides. C'est pour quoi, dans la description et les revendications qui suivent, les mots « grain et grains » désignent les grains et graines, mais aussi tous matériaux partiellement ou totalement vitreux, ou translucides et dont il faut connaitre une caractéristique au cœur du grain.

Actuellement, pour l'observation de la vitrosité, on procède généralement selon deux méthodes connues, dont l'une consiste à utiliser un éclairage par le dessus éclairant les grains à analyser disposés sur un fond blanc, ou bleu pour le riz, et l'autre un éclairage par dessous.

Avec l'éclairage par-dessus, on évite ainsi le fond noir absorbant qui créé trop de contraste, qui fatigue les yeux et qui a tendance à retenir les poussières et à se salir.

La vitrosité se révèle par la lumière qui se réfléchit sur le fond et retraverse le grain en direction de l'œil de l'observateur, ou de l'objectif d'une caméra, mais ce type d'éclairage ne peut être uniforme sur toute la surface du grain. On comprend alors pourquoi le fond n'est pas adapté et révèle moins la vitrosité.

L'autre technique consiste à utiliser un arrière plan lumineux, éclairant par dessous et généralement équipé d'un verre dépoli pour constituer un fond homogène. La vitrosité se révèle par la lumière qui traverse le grain en direction de l'œil de l'observateur ou de l'objectif d'une caméra.

Selon ces deux techniques, pour mieux voir le grain et sa vitrosité intérieure, il faut augmenter la puissance de l'éclairage. Or, cet accroissement de lumière augmente l'éclairement de l'arrière plan, et créé en retour vers les moyens d'observation, un éblouissement qui est préjudiciable au confort visuel et n'améliore pas le contraste.

L'objet de l'invention est de fournir des moyens complémentaires permettant d'augmenter le contraste de lecture, sans créer par ailleurs d'éblouissement, et en révélant au maximum la vitrosité du grain observé sur l'arrière plan.

On connait par le document US5 835 206 une unité d'analyse de la vitrosité des grains comprenant un bâti porteur d'une plaque support horizontale recevant les grains à analyser et éclairée par des moyens d'éclairage, définissant un axe optique X'-X, une fenêtre d'observation définissant une axe observation Z'-Z orthogonal à elle, et, éventuellement, une caméra d'enregistrement de la séquence d'analyse. Les moyens d'éclairage traversent un film de polarisation disposé au dessus d'eux, tandis qu'une fenêtre d'observation comprend un hublot, et, au dessous de ce hublot, un écran de polarisation, de direction croisée avec celle du film de polarisation associé à la plaque.

En pratique, le caractère diffus de la lumière produite par les moyens d'éclairage ne permet pas d'obtenir un contraste suffisant.

L'objet de l'invention est de fournir des moyens complémentaires permettant d'augmenter le contraste de lecture, sans créer par ailleurs d'éblouissement, et en révélant au maximum la vitrosité du grain observé sur l'arrière plan.

Selon l'invention, et en complément de ce qui précède, l'axe d'observation Z'-Z et le hublot de la fenêtre d'observation sont inclinés par rapport à l'axe optique X'-X, de manière que l'œil de l'observateur ou le moyen d'observation, ne soit pas dans l'axe optique de la source lumineuse, tandis que le projecteur est associé à des moyens assurant la collimation de son faisceau lumineux.

Avec cet agencement, les rayons lumineux sont rendus parallèles et le contraste est augmenté, puisque la polarisation est meilleure et l'analyseur intervient mieux sur la lumière incidente. Les grains sont plus lumineux sur un fond plus sombre, différent de l'arrière plan lumineux obtenu avec une source étendue.

De plus, le désaxage de l'observation améliore encore ce contraste en le rendant plus régulier, en éliminant la surbrillance de la source, consécutive à sa traversée des polariseurs qui n'ont pas un pouvoir polarisant à 100%, mais plutôt à 95%. Ainsi, l'observateur voit la lumière diffusée par la vitrosité des grains, sans percevoir d'aucune manière la source lumineuse elle-même.

Avec cette configuration optique :
- les grains sont extrêmement lumineux, car éclairés par le dessous, ils transmettent d'autant plus de lumière qu'ils sont vitreux, et que l'observateur distingue très bien cette lumière diffuse ;
- l'arrière plan est sombre, car la lumière incidente polarisée est supprimée, atténuée ou coupée par l'analyseur de polarisation croisée.

On obtient ainsi un contraste considérablement plus élevé que par les autres moyens habituels cités plus haut. Cet équipement permet aussi de « voir à l'intérieur du grain », si son enveloppe n'est pas trop opaque. Par exemple, voir si l'intérieur d'un grain de paddy est rouge ou vert.

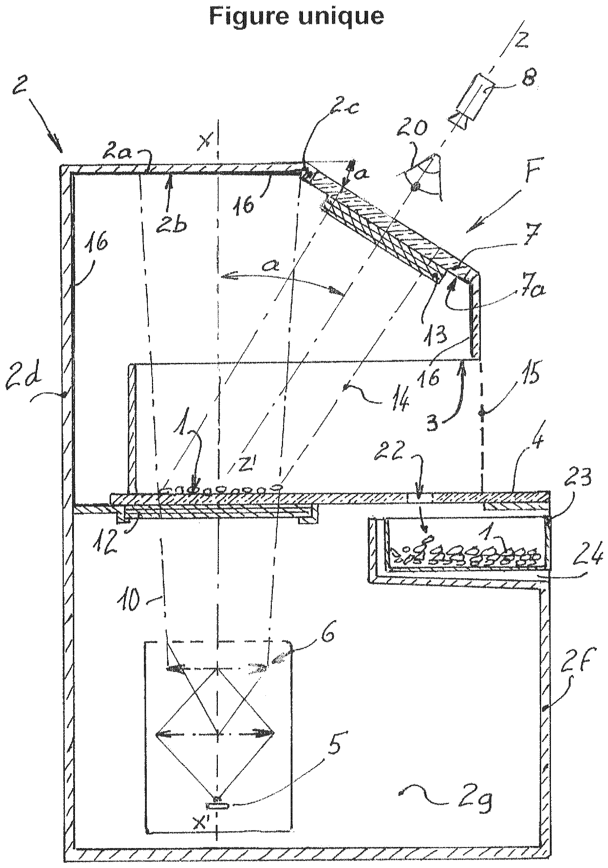

D'autres caractéristiques et avantages ressortiront de la description qui suit, en référence au dessin schématique annexé, dont l'unique figure représente, une vue de coté en coupe verticale d'une unité de laboratoire avec alimentation manuelle et tri manuel des grains.

Dans la forme d'exécution représentée, la référence numérique 2 désigne le bâti de l'appareil, comportant une échancrure transversale 3. Celle-ci forme un accès à une plaque support 4 qui, en matériau translucide ou transparent, non polarisable, et par exemple en verre, est apte à recevoir les grains 1 à analyser. Cette plaque est avantageusement traitée antireflet et munie de rainures parallèles de positionnement des grains.

La référence 5 désigne un projecteur et 6 son dispositif optique concentrant son faisceau lumineux 10 émis suivant l'axe optique X'-X, tandis que 7 désigne le hublot d'une fenêtre d'observation F. Ce hublot est orthogonal à l'axe d'observation Z'-Z allant à l'œil 20 de l'observateur, ou à une caméra vidéo 8, raccordée à un enregistreur et à une unité de traitement, non représentés.

Selon l'invention, le projecteur 5 est disposé au dessous de la plaque 4 et envoie son faisceau 10 sensiblement verticalement jusqu'en direction du toit 2a du bâti 2. Avant de traverser par-dessous la plaque support 4, le faisceau 10 traverse un film de polarisation 12, disposé contre et sous cette plaque.

Dans cette forme d'exécution, le hublot 7 est porté par une partie 2c du bâti 2, qui est inclinée par rapport au toit 2a d'une valeur a, comprise entre 10 et 70 degrés et par exemple de 30 degrés. L'axe d'observation Z'-Z qui traverse orthogonalement le hublot est incliné du même angle **a** par rapport à l'axe optique X'-X.

Le hublot 7 est en matériau translucide ou transparent non polarisable et par exemple en verre. Il présente une face interne 7a contre laquelle est appliqué un écran analyseur 13 assurant la polarisation de la lumière incidente 14, renvoyée par les grains, cette polarisation étant croisée, de préférence à 90 degrés, avec la polarisation du faisceau lumineux 10 fournie par le film 12.

Pour procéder à une analyse, l'analyste dépose une poignée de grains 1 sur le support 2 et allume le projecteur 5. Le faisceau lumineux émis 10 est polarisé par le film 12, puis traverse le support 4 et les grains 1 qui reposent sur lui, avant de venir contre face 2b du toit 2.

Quand l'observateur 20 observe les grains 1 à travers le hublot 7 et l'écran 13, la lumière incidente 14, qui devrait être perçue en totalité au poste d'observation, est coupée ou au moins amoindrie par l'écran analyseur 13. Il en résulte que la lumière incidente ne gène pas l'observateur, ne l'éblouit pas et opacifie le fond, afin qu'il perçoive par contraste d'autant mieux les grains. Les grains vitreux lui apparaissent d'autant plus lumineux qu'ils sont éclairés par le dessous.

De préférence et pour obtenir un meilleur contraste, les moyens optiques 6 associés au projecteur 5 assurent la collimation du faisceau lumineux 10. Les rayons lumineux sont ainsi rendus parallèles, procurent de meilleures polarisations et un meilleur assombrissement du fond à travers l'analyseur 13.

On notera que le décalage angulaire a entre l'axe optique X'-X et l'axe d'observation Z'-Z permet d'éviter que les moyens d'observation, œil 20 ou caméra 8, soient éblouis par la forte puissance de la source lumineuse collimatée qui parviendrait à traverser le film polariseur 12 et l'écran 13. Grâce à ce décalage angulaire l'observateur ne perçoit que la lumière diffusée par la vitrosité des grains, sans être gêné par la source lumineuse d'origine.

Le traitement antireflets de la plaque 4 permet d'éviter le reflet des grains et procure des images plus faciles à analyser.

L'unité d'analyse comprend une loupe qui, disposée au-dessus de la fenêtre d'observation, assure un grossissement de l'image.

De façon connue, pour protéger les conditions d'observation des grains 1 de toute pollution lumineuse extérieure, le bâti 2 est capoté, dans ses parties contenant les moyens d'éclairage 5, les moyens optiques 6 et dans sa partie 2c portant le support 7, par des parois verticales, respectivement, arrière 2d, avant 2f et latérales 2g.

Dans le même bût, son échancrure 3, permettant l'accès manuel à la plaque support 4, est bordée par des rideaux noirs écartables 15 et toutes ses surfaces internes pouvant réfléchir la lumière ou un reflet sont recouvertes par un revêtement absorbant la lumière, par exemple une peinture de couleur noire 16.

Dans une réalisation et pour un meilleur confort d'observation, la fenêtre d'observation F est protégée par un capotage, éventuellement amovible, de couleur foncée, par exemple bleue ou noire mate, évitant les reflets de la lumière extérieure sur le hublot d'observation 7.

Par ailleurs, si la lumière émise par le projecteur 5 est en générale blanche et polychromatique pour l'analyse de la vitrosité de grains, pour analyser d'autres caractéristiques de grains ou d'autres produits vitreux, elle peut être de longueurs d'ondes déterminées par l'application et par exemple être une lumière monochromatique, une lumière infrarouge ou une lumière dans l'ultraviolet.

Cette unité d'analyse de la vitrosité peut être utilisée avec une alimentation manuelle des grains 1 qui, pour faciliter l'observation, sont posés sur la plaque support 4. Après tri, les grains sont dirigés manuellement vers au moins un trou d'évacuation 22 qui permet de les évacuer dans un réceptacle 23 disposé dans une niche 24 du bâti et ménagée sous la plaque support 4.

En pratique, la plaque 4 comporte autant de trous 22 et de réceptacles 23, que le tri sélectionne des catégories de grains, par exemple grains sains, grains mouchetés, grains mitadinés, grains piqués......

Dans une variante de réalisation, la plaque support 4 recevant les grains est constituée par une vitre de polarisation.

L'unité d'analyse peut aussi faire partie d'une installation automatique assurant une observation avec analyse continue et comprenant des moyens automatiques d'alimentation des grains sur une plaque support fixe, ou mobile, des moyens d'observation avec caméra 8 et unité de reconnaissance de défauts et/ou de vitrosité, des moyens de répartition des types de grains en direction des trous d'éjection 22 de la plaque 4, ces moyens réagissant aux signaux de l'unité de reconnaissance, et des moyens de distribution des grains triés vers différents lieux de stockage affectés à leur catégorie, par exemple grains sains vitreux, grains mouchetés, grains mitadinés, grains piqués......

Dans une autre réalisation, non représentée, les grains à analyser sont déversés sur un plan incliné sur lequel leur image est prélevée à la volée, puis analysée par un dispositif de traitement d'image réagissant sur un dispositif de triage par soufflettes.

Dans une autre forme de réalisation, l'unité d'analyse comprend, au dessous et à coté du projecteur 5 une deuxième caméra qui permet l'observation du grain en mode « réflexion » et non plus seulement en mode « transmission-diffusion ». Cela permet avec la même unité d'analyse d'obtenir des vues en transparence, par « transmission-diffusion » et en réflexion par dessous.

Il ressort de cette description que la combinaison des différents composants de l'unité d'analyse permet bien, de façon manuelle ou automatique, de faciliter l'observation et l'analyse de grains et graines, au moins partiellement vitreux, en utilisant au mieux leur vitrosité sur un fond opaque et en supprimant les causes d'éblouissement par la lumière incidente.

## Revendications

1. Unité d'analyse de la vitrosité de grains non complètement opaques, comprenant un bâti (2) porteur d'une plaque support horizontale (4), en matériau translucide ou transparent, recevant les grains (1) à analyser, des moyens (5) d'éclairage de la plaque (4) situés sous ladite plaque définissant un axe optique X'-X, une fenêtre d'observation F définissant un axe d'observation Z'-Z orthogonal à elle, et, éventuellement, une caméra d'enregistrement (8) de la séquence d'analyse, unité dans laquelle le faisceau lumineux (10) des moyens d'éclairage (5) traverse un film de polarisation (12) disposé au dessus d'eux, tandis que la fenêtre d'observation comprend un hublot (7), en matériau transparent non polarisable et, au dessous de cet hublot, un écran (13) de polarisation, de direction croisée par rapport à la direction du film de polarisation (12), **caractérisée en ce que** l'axe d'observation Z'-Z et le hublot (7) de la fenêtre d'observation sont inclinés par rapport à l'axe optique X'-X du faisceau lumineux (10), de manière que l'œil (20) de l'observateur ou la caméra d'enregistrement (8), ne soit pas dans l'axe optique de la source lumineuse, tandis que les moyens d'éclairage sont constitués par un projecteur (5) associé à des moyens assurant la collimation de son faisceau lumineux (10).

2. Unité d'analyse de la vitrosité de grains non complètement opaques selon la revendication 1, **caractérisée en ce que** l'axe d'observation Z'-Z est incliné par rapport à l'axe optique X'-X d'un angle a compris entre 10 et 70 degrés.

3. Unité d'analyse de la vitrosité de grains non complètement opaques selon la revendication 1, **caractérisée en ce que** la plaque support (4) est en verre traité antireflets.

4. Unité d'analyse de la vitrosité de grains non complètement opaques selon la revendication 1, **caractérisée en ce que** la plaque support (4) est munie de rainures parallèles de positionnement des grains.

5. Unité d'analyse de la vitrosité de grains non complètement opaques selon la revendication 1 **caractérisée en ce que** le bâti (2) est capoté dans ses parties contenant les moyens optiques et les moyens d'éclairage, par des parois (2a, 2c, 2d, et 2f), et est bordé, dans sa partie munie d'échancrures latérales (3) permettant l'accès manuel à la plaque support (4), par des rideaux (15) de protection contre les lumières parasites extérieures.

6. Unité d'analyse de la vitrosité de grains non complètement opaques selon la revendication 1 **caractérisée en ce qu'**elle comprend une loupe disposée au-dessus de la fenêtre d'observation et assurant un grossissement de l'image.

7. Unité d'analyse de la vitrosité de grains non complètement opaques selon la revendication 1 **caractérisée en ce que** la plaque support (4) recevant les grains est constituée par une vitre de polarisation.

8. Unité d'analyse de la vitrosité de grains non complètement opaques selon la revendication 1 **caractérisée en ce qu'**elle comprend, au dessous et à coté du projecteur (5), une deuxième caméra qui permet l'observation du grain en mode « réflexion» et non plus seulement en mode « transmission-diffusion ».

9. Installation automatique assurant une observation avec analyse continue **caractérisée en ce qu'**elle comprend une unité d'analyse de la vitrosité de grains non complètement opaques selon la revendication 1 et l'une quelconque des revendications 2 à 7, l'unité d'analyse de la vitrosité comprenant des moyens d'observation avec ladite caméra (8) et une unité de reconnaissance de défauts et de vitrosité, l'installation automatique comprenant des moyens automatiques d'alimentation des grains sur la plaque support fixe, des moyens de répartition des types de grains en direction des trous d'éjection (22) de la plaque (4), ces moyens réagissant aux signaux de l'unité de reconnaissance, et des moyens de distribution des grains triés vers différents lieux de stockage affectés à leur catégorie, par exemple grains sains vitreux, grains mouchetés, grains mitadinés, grains piqués.

## Patentansprüche

1. Analyseeinheit für die Glasigkeit nicht vollständig opaker Körner, mit einem Rahmen (2), der eine horizontale Trägerplatte (4) aus durchscheinendem oder transparentem Material enthält, zur Aufnahme der zu analysierenden Körner (1), Mittel zur Beleuchtung (5) der Platte (4), die unter dieser Platte angeordnet sind und die eine optische Achse X'-X definieren, ein Beobachtungsfenster F, das eine Beobachtungsachse Z'-Z definiert, die zu ihm orthogonal ist,
und eventuell eine Aufzeichnungskamera (8) für die Analysesequenz, in dieser Einheit geht der Lichtstrahl (10) der Beleuchtungsmittel (5) durch eine Polarisationsfolie (12) hindurch, die über ihnen angeordnet ist, während das Beobachtungsfenster ein Sichtfenster (7), aus transparentem, nicht polarisierbarem Material enthält und unter diesem Sichtfenster, einen Polarisationsschirm (13), mit einer Richtung, die die Richtung der Polarisationsfolie (12) kreuzt,
**dadurch gekennzeichnet, dass die** Beobachtungsachse Z'-Z und das Sichtfenster (7) des Beobachtungsfensters bezogen auf die optische Achse X'-X des Lichtstrahls (10) geneigt sind, so dass das Auge (20) des Beobachters oder die Aufzeichnungskamera (8) sich nicht in der optische Achse der Lichtquelle befinden, während die Beleuchtungsmittel aus einem Projektor (5) bestehen, verbunden mit Mitteln zur Sicherstellung der Kollimation seines Lichtstrahls (10).

2. Analyseeinheit für die Glasigkeit nicht vollständig opaker Körner, nach Anspruch 1, **dadurch gekennzeichnet, dass** die Beobachtungsachse Z'-Z bezogen auf die optische Achse X'-X um einen Winkel zwischen 10 und 70 Grad geneigt ist.

3. Analyseeinheit für die Glasigkeit nicht vollständig opaker Körner, nach Anspruch 1, **dadurch gekennzeichnet, dass** die Trägerplatte (4) aus entspiegeltem Glas besteht.

4. Analyseeinheit für die Glasigkeit nicht vollständig opaker Körner, nach Anspruch 1, **dadurch gekennzeichnet, dass** die Trägerplatte (4) mit parallelen Rillen zum Positionieren der Körner versehen ist.

5. Analyseeinheit für die Glasigkeit nicht vollständig opaker Körner, nach Anspruch 1 **dadurch gekennzeichnet, dass** der Rahmen (2) in seinen Teilen, die die optischen und die Beleuchtungsmittel enthalten, durch Wände (2a, 2c, 2d und 2f) abgedeckt ist und in seinem Teil mit seitlichen Einschnitte (3), die den manuellen Zugang zur Trägerplatte (4) ermöglichen, über Schutzvorhänge (15) gegen Streulicht von außen verfügt.

6. Analyseeinheit für die Glasigkeit nicht vollständig opaker Körner, nach Anspruch 1, **dadurch gekennzeichnet, dass** sie eine Lupe umfasst, die über dem Beobachtungsfenster angeordnet ist und eine Vergrößerung des Bildes gewährleistet.

7. Analyseeinheit für die Glasigkeit nicht vollständig opaker Körner, nach Anspruch 1, **dadurch gekennzeichnet, dass** die Trägerplatte (4) zur Aufnahme der Körner aus einer Polarisationsscheibe besteht.

8. Analyseeinheit für die Glasigkeit nicht vollständig opaker Körner, nach Anspruch 1, **dadurch gekennzeichnet, dass** sie unter und neben dem Projektor (5), eine zweite Kamera enthält, mit der das Korn im Modus « Reflektion » beobachtet werden kann und nicht nur im Modus « Transmission- Diffusion ».

9. Automatische Anlage zur Gewährleistung einer Beobachtung mit stetiger Analyse, **dadurch gekennzeichnet, dass** sie eine Analyseeinheit für die Glasigkeit nicht vollständig opaker Körner nach Anspruch 1 und irgendeinem der Ansprüche 2 bis 7 enthält, wobei die Analyseeinheit für die Glasigkeit Mittel zur Beobachtung mit dieser Kamera (8) enthält sowie eine Einheit zur Erkennung von Fehlern und der Glasigkeit, die automatische Anlage enthält Mittel zur automatischen Körnerzufuhr auf einer festen Trägerplatte, Mittel zur Verteilung der Körnertypen in Richtung der Auswerflöcher (22) der Platte (4), wobei diese Mittel auf die Signale der Erkennungseinheit reagieren sowie Mittel zur Verteilung der sortierten Körner auf verschiedene, für die jeweilige Klasse vorgesehene Lagerorte, zum Beispiel gesunde, glasige Körner, fleckige Körner, Körner, die ihr glasiges Aussehen ganz oder teilweise verloren haben, beschädigte Körner.

## Claims

1. Unit for analyzing the vitreousness of partially opaque grains, comprising a frame (2) carrying a horizontal support plate (4), made of translucent or transparent material, receiving the grains (1) to be analyzed, illuminating means (5) to illuminate the plate (4) located under said plate defining an optical axis X'-X, an observation window F defining an observation axis Z'-Z orthogonal thereto, and, optionally, a recording camera (8) for the analysis sequence, wherein the light beam (10) of the illuminating means (5) passes through a polarization film (12) positioned above them, while the observation window comprises a viewport (7), made of non-polarizable transparent material and, below this viewport, a polarization screen (13), in cross-wise direction with respect to the direction of the polarization film (12),
**characterized in that** the observation axis Z'-Z and the viewport (7) of the observation window are angled with respect to the optical axis X'-X of the light beam (10), in such a way that the eye (20) of the observer or the recording camera (8) is not aligned with the optical axis of the light source, while the illuminating means consist of a projector (5) associated with means ensuring the collimation of its light beam (10).

2. Unit for analyzing the vitreousness of partially opaque grains according to claim 1, **characterized in that** the observation axis Z'-Z is angled with respect to the optical axis X'-X by an angle a in the range from 10 to 70 degrees.

3. Unit for analyzing the vitreousness of partially opaque grains according to claim 1, **characterized in that** the support plate (4) is anti-reflective treated glass.

4. Unit for analyzing the vitreousness of partially opaque grains according to claim 1, **characterized in that** the support plate (4) is provided with parallel grooves for positioning the grains.

5. Unit for analyzing the vitreousness of partially opaque grains according to claim 1, **characterized in that** the frame (2) is covered by walls (2a, 2c, 2d, and 2f) relative to its parts containing the optical means and the illuminating means, and is bordered by curtains (15) for protection against external parasitic lights relative to its part provided with lateral notches (3) allowing manual access to the support plate (4).

6. Unit for analyzing the vitreousness of partially opaque grains according to claim 1, **characterized in that** it comprises a magnifying glass disposed above the observation window and ensuring an enlargement of the image.

7. Unit for analyzing the vitreousness of partially opaque grains according to claim 1, **characterized in that** the support plate (4) receiving the grains is constituted by a polarization window.

8. Unit for analyzing the vitreousness of partially opaque grains according to claim 1, **characterized in that** it comprises, below and beside the projector (5), a second camera which allows the observation of the grain in "reflection" mode and not only in "transmission-diffusion" mode.

9. Automatic installation ensuring observation with continuous analysis, **characterized in that** it comprises a unit for analyzing the vitreousness partially opaque grains according to claim 1 and any one of claims 2 to 7,
the unit for analyzing the vitreousness comprising observation means with said camera (8) and a unit for recognizing defects and a vitreousness, the automatic installation comprising automatic means for feeding the grains onto a fixed support plate,
means for distributing the types of grains in the direction of the ejection holes (22) of the plate (4), which means are responsive to the signals of the recognition unit, and means for distributing the sorted grains to different storage locations assigned to their category, for example vitreous healthy grains, mottled grains, starchy grains, stung grains.
